# EUROPEAN PATENT SPECIFICATION

(11) **EP 2 144 589 B1**
(45) Date of publication and mention of the grant of the patent: **27.07.2016**
(21) Application number: 07748474.9
(22) Date of filing: 08.05.2007
(51) Int. Cl.: A61J 1/20, A61M 5/32

(54) **FLUID TRANSFER DEVICE**
FLÜSSIGKEITSTRANSFERVORRICHTUNG
DISPOSITIF DE TRANSFERT DE FLUIDE

(43) Date of publication of application: 20.01.2010
(73) Proprietor: Carmel Pharma AB, 402 28 Göteborg (SE)
(72) Inventor: HORPPU, Petri, S-114 50 Stockholm (SE); ELLSTRÖM, Anna, S-413 25 Göteborg (SE)
(74) Representative: Winqvist, Jens Pontus
(86) International application number: PCT/SE2007/050313
(87) International publication number: WO 2008/136720

(56) References cited:
- EP-A1- 0 475 789
- EP-A1- 1 731 128
- WO-A2-00/15292
- US-A- 3 390 677
- US-A- 4 898 209
- US-A- 6 071 270

## Description

### TECHNICAL FIELD

The present invention relates to a fluid transfer device and more specifically a piercing member protection device arranged with a first and a second fluid container.

### BACKGROUND OF THE INVENTION

A serious problem in connection with drug preparation, drug administration and other similar handling is the risk that medical and pharmacological staff are exposed to drugs or solvents which might escape into the ambient air. Medical and pharmacological staff are also frequently exposed to needles, syringes and piercing members in their daily work. Such exposure may lead to accidents such as the staff piercing or scratching themselves. This problem is particularly serious when cytotoxins, antiviral drugs, antibiotics and radiopharmaceuticals are concerned. Other hazardous areas may be sampling taking such as samples concerning virus infections or the like.

For this reason, there has been a need of safer systems for handling and administrating drugs and other medical substances.

Accordingly, U. S. Patent No. 4,564, 054 (Gustavsson) discloses a fluid transfer device for transferring a substance from one vessel to another vessel while avoiding leakage of liquid and gas contaminants by protecting the piercing member. The disclosed device comprises a first member designed as a hollow sleeve and having a piercing member provided with a passageway. The piercing member is attached to the first member which has a first barrier member at one end just opposite the tip of the piercing member. Thereby, the piercing member can be passed and retracted through the first barrier member which seals one end of the first member. The fluid transfer device further comprises a second member which is attached to or attachable to one of the vessels or to means arranged to communicate therewith. The second member has a second barrier member, and mating connection means arranged on the first and second members for providing a releasable locking of the members with respect to each other. The barrier members are liquid and gas-proof sealing members which seal tightly after penetration and retraction of the piercing member and prevent leakage of liquid as well as gas contaminants. In the connected position of the first and second members, the barrier members are located in such a way with respect to each other that the piercing member can be passed therethrough.
US 5,170,88 discloses a container defining a first compartment and another compartment and means of separation of the two; a perforator assembly and means for displacing the perforating unit.

When performing infusion, it is often necessary to inject a drug or other medical substance into the infusion fluid inside an infusion bag or other infusion fluid container. This is often done by means of penetrating a septum or other fluid barrier of an injection port on the infusion bag or on the infusion fluid line with a needle of a syringe filled with the medical fluid in question. However, even before this it may be necessary to transfer the medical fluid from a vial to a syringe and then from the syringe to a secondary container.

In order to transfer a fluid, a first and a second fluid container is connected to a fluid transfer device. Such first and second fluid containers may be e.g. a vial and a syringe. In special cases the fluid transfer device may be in the form of a piercing member protection device to protect a piercing member. However, it has been found that some transfer devices lock the fluid containers with respect to each other in a non favourable position after assembly. Such a position may result in difficulties in e.g. reading the volume indication on at least one of the fluid containers. In attempts to eliminate this unfavourable position by e.g. turning the whole fluid transfer device it has surprisingly been found that such attempts cause an additional hazardous step for users e.g. medical staff when transferring hazardous fluids. If a syringe, vial or infusion system or the like is attached, the user's glove may e.g. be caught in the transfer device during turning of the syringe. Usually complications like this may lead to the gloves being torn. This may in turn lead to exposure to contaminants for the user. Further, contaminants may escape from the transfer device during the turning of the syringe. Even worse the connection means may be detached during such turning. In this worst case scenario, the piercing member is usually in its unsecured position and the user is very likely to be exposed to the sharp tip of the piercing member and the hazardous fluid. A further disadvantage is that one fluid container can be accidentally disconnected during use. A disconnection during use may expose not only a piercing member to a user but also expose the user to the hazardous fluid.

### SUMMARY OF THE INVENTION

It is therefore an object of the present invention to provide a fluid transfer device such as a piercing member protection device which partly or fully reduces the risk of complications during turning of the device or parts attached thereto. The present invention at least partly solves this problem by providing a fluid transfer device such as a piercing member protection device for transferring fluid from a first fluid container to a second fluid container, as defined by claim 1. The fluid transfer device comprises a longitudinal axis A, a first connection part comprising connection means, i.e. a connector, for connecting to the first fluid container. The fluid transfer device further comprises a second connection part rotatably mounted to said first connection part. The second connection part further comprises connection means, i.e. a threaded coupling, for connecting the second fluid container. The present invention allows any fluid container attached to the fluid transfer device to be turned easily and safely without complications.

The fluid transfer device is further arranged with a first locking means, i.e. at least one protrusion, wherein the first locking means substantially prevents the first connection part from turning in a predetermined direction with respect to the second connection part while still allowing it turn in a direction opposite of the predetermined direction. The mentioned embodiment has several advantages. One advantage is that the fluid transfer device may be attached to a first fluid container by a turning motion (in the predetermined direction) without using the turning function between the first and second connection part. This will also have the advantage of preventing the fluid transfer device from being unscrewed after attachment with the first fluid container since any turning in the direction opposite of the predetermined direction will only result in the turning between the first and second connection part of the fluid transfer device due to the first locking means.

According to the present invention the fluid transfer device is a piercing member protection device. The piercing member protection device may comprise a piercing member protection part having a protection chamber to protect a piercing member. The piercing member protection device has a secured position, in which at least the tip of said piercing member is enclosed within the protection chamber of the piercing member protection part, so as to prevent the tip of the piercing member from exposure. The piercing member protection device further has an unsecured position, in which the tip of the piercing member is arranged outside the protection chamber of said piercing member protection part.

Preferably the first locking means on the fluid transfer device enables at least a 90° turn, preferably at least a 180° turn, more preferably at least a 270° turn, or even more preferably at least a 360° turn of the second connection part. The latter first locking means has the advantage of letting an attached second fluid container be turned fully around, giving the user full access to any indications present on the surface of the second fluid container. In one embodiment of the present invention the first locking means connects the first and second connection parts by means of a locking groove and a locking protrusion, such a locking groove is preferably arranged transversally to the longitudinal axis A around the periphery of the first connection part while the locking protrusion is preferably arranged on the second connection part. In an alternative embodiment of the present invention the locking protrusion may be arranged on the second connection part of the fluid transfer device while the locking groove is arranged on the first connection part. In the cases when at least a 90°, 180° or a 270° turn of the fluid transfer device is preferable, the first locking means connects to the first and second connection part by means of a locking groove arranged transversally to the longitudinal axis A around at least a part of the periphery of the connection part.

The locking protrusion may by in the form of a saw tooth element, i.e. a ratchet, which interacts with the locking groove. Preferably there are between 1 to 30 saw tooth elements, preferably 2 to 10 or even more preferably 2 to 5 saw tooth elements. The saw tooth elements are preferably arranged at an angle between 2 to 15°, preferably 4 to 12° more preferably 5 to 10° with respect to an axis perpendicular to the longitudinal axis A. Such angle allows for a smooth turning in one direction while still enabling an efficient prevention of turning in the predetermined direction.

In one embodiment of the present invention, the fluid transfer device is equipped with the mentioned piercing member in order to transfer fluid from one fluid container to a second fluid container. This embodiment of the present invention is advantageous because it reduces the amount of occasions in which a user may be exposed to a piercing member. The piercing member might however be arranged on one of the fluid containers which is arranged for connection with the fluid transfer device in order to transfer fluid from the first fluid container to the second fluid container. The fluid transfer device may further comprise a stabilization member arranged to stabilize or guide the piercing member. Stabilization of the piercing member, regardless of whether it is arranged on the second connection part or an attachable fluid container, is important to prevent the piercing member from breaking or bending. Preferably such a stabilization member comprises a hollow tube arranged substantially along the longitudinal axis A or parallel thereto, wherein the hollow tube is arranged to at least partly enclose the piercing member. In cases were the piercing member is arranged on a fluid container the stabilization member at least partly enclose the piercing member after assembly.

The fluid transfer device according to the present invention may optionally be used in various different fields of technology such as food manufacturing technology or medical technology (preferred field of technology). In the embodiments when the fluid transfer device is a piercing member protection device or a fluid transfer connector, it is preferably a medical piercing member protection device or medical fluid transfer connector.

### DEFINITIONS

With the term "piercing member" it is meant a substantially hollow object, such as a needle like tube or a needle, which may pierce a membrane or similar in order to withdraw or infuse a gas fluid, a liquid fluid or a mixture thereof (i.e. a fluid). The mentioned membrane may be the skin of a patient or a flexible barrier member on e.g. a vial or on an infusion bag or the like.

With the term "medical" piercing member protection device or "medical" fluid transfer device is meant a device which is used directly or indirectly in the medical field of technology e.g. in hospital environments or hospital like environments, pharmaceutical industry, home care etc. Examples of medical devices are needles, needle like tubes, syringes, infusion bags, medical fluid transfer devices, medical vials, medical fluid containers, medical sampling containers or the like.

With the term "interacting protrusion element" is meant at least one element which interacts with at least one part of the first locking means when said first locking means is in the form of a locking protrusion or a saw tooth arrangement as described. Although a saw tooth may interact with a protrusion, it can likewise interact with a groove. The "interacting protrusion element" is then considered to be the material which fully or partly defines that groove (e.g. the wall of the groove).

### BRIEF DESCRIPTION OF THE DRAWINGS

Fig. 1 shows a cross section of a fluid transfer device in the form of a piercing member protection device according to the present invention illustrated in its secured position.
Fig. 2 shows a cross section of the fluid transfer device according to the present invention in fig. 1 as seen from a slightly different angle. The piercing member protection device is in its unsecured position.
Fig. 3a shows a cross section of a fluid transfer device according to the present invention.
Fig. 3b shows a front view of a fluid transfer device according to the present invention.
Fig. 3c shows a side view of a fluid transfer device according to the present invention.
Fig. 4 shows a fluid transfer device according to the present invention as seen in perspective.
Fig. 5 shows a cross section of the fluid transfer device in fig. 4.

### DETAILED DESCRIPTION OF PREFERRED EMBODIMENTS

Generally dependent upon how the first and the second connection part are arranged to interact via the first locking means and the mounting means, the fluid transfer device may display the following functions:
1) The first locking means may be arranged so as to prevent the turning of the first connection part with respect to the second connection part in a predetermined direction. In this embodiment the first locking means is always engaged, no play exists between the first and the second connection part. As an example, a saw tooth protrusion and an interacting protrusion element will always be engaged.
2) The first locking means may be arranged so as to prevent the turning of the first connection part with respect to the second connection part in a predetermined direction and one position in which the first connection part may be turned in any direction with respect to the second connection part. This embodiment has a play between the first connection part and the second connection part so that e.g. a saw tooth protrusion and an interacting protrusion element will be able to disengage.
3) The first locking means may be arranged so as to prevent the turning of the first connection part with respect to the second connection part in a predetermined direction during assembly with a fluid container and one position in which the first connection part may, after assembly, be turned in any direction with respect to the second connection part and whereby the first locking means can not again be engaged after disengagement. This embodiment, which actually is another embodiment of the embodiment listed under point 2, can be achieved by suitably adapt the size of the play with respect to the length of the second connection part and the mounting means.

These embodiments will be further described in greater detail with the aid of the following examples:

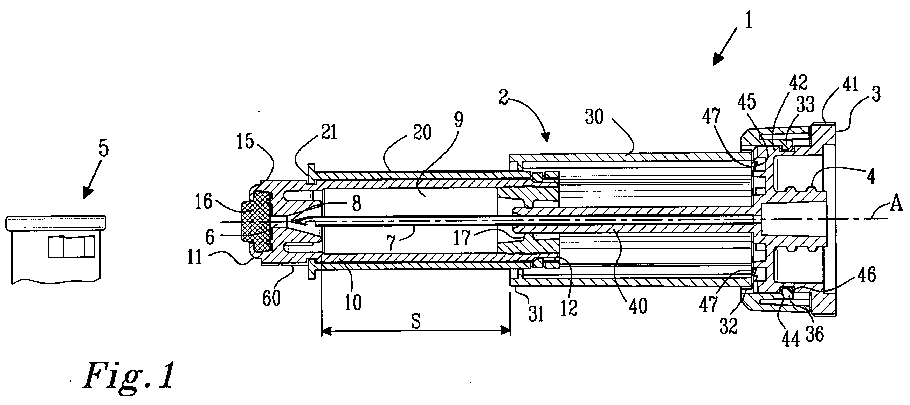

In Fig. 1 a fluid transfer device according to the present invention in the form of a piercing member protection device 1 is illustrated having a first connection part 2 in the form of a piercing member protection part 2 which is rotably mounted to a second connection part 3. The piercing member protection part 2 is in fig. 1 shown in its secured position.

The piercing member protection device 1 comprises a longitudinal axis A. The piercing member protection part 2 comprises a first member 10, a second member 20 and a third member 30. The first member 10 has a first end 11 and a second end 12, the first end 11 comprises connection means 15 for connecting to a first container 5. The second member 20 at least partly encloses the first member 10 and the third member 30 at least partly encloses the first 10 and the second member 20. The third member 30 has a first end 31 and a second end 32. The second end 32 of the third member 30 comprises means for attaching the third member to the second connection part 3.

The first end 11 of the first member 10 also comprises an injection port 6 through which at least a part of a piercing member 7 is to be guided through to the first container 5 when connected thereto, i.e. when the piercing member protection device goes from the secured position to the unsecured position. When the piercing member protection part 2 is in the secured position, at least the tip 8 of the piercing member 7 is fully enclosed inside the first member 10 so as to prevent the sharp tip 8 of the piercing member 7 coming in contact with a user. In this embodiment of the present invention the first member 10 defines a protection chamber for the tip of the piercing member 7. The piercing member 7 may either be attached, as described below to the fluid transfer device, or to a separate device, such as a fluid container, which can be attached to the fluid transfer device 3.

The first, second and third members 10, 20, 30 can be manufactured from any suitable material but they preferably comprise a thermoplastic material such as polypropylene, polyethylene, polyurethane, polystyrene, polyoxymethylene, acrylonitrile-butadienestyrene copolymer (ABS), polyethylene terephthalate or mixtures thereof. The first, second and third members 10, 20, 30 can be made of different materials or of the same material. In one embodiment, the first member 10 is made of a transparent material in order to allow the user of the device to easily see if proper function is achieved. A suitable material should be somewhat flexible to allow for the second member 20 to be threaded onto the first member 10 without major difficulties but rigid enough to provide enough protection for the piercing member arranged inside the first member 10 when such is present.

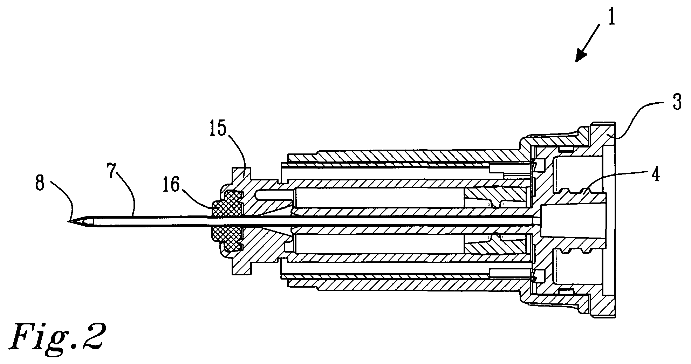

The third member 30 may slide along the longitudinal axis A from a secured position (as shown in fig. 1) to an unsecured position (as shown in fig. 2). It is noted that an unsecured position is achieved somewhere along the longitudinal axis A dependent on the length of the piercing member 7. Preferably, the third member 30 is moved a minimum length of 10 % of the total length (i.e. the total length being the maximum length possible to move the third member 30) before the fluid transfer device is in its unsecured position. The total length is illustrated in fig. 1 with a slide arrow S. The third member 30 may further be turned with respect to the second member 20 from a locked position to an unlocked position. When the piercing member protection device is in its unsecured position, a fluid communication is preferably provided between the first and the second container, while in its secured position, no fluid communication is provided between the first and the second container. The main difference is however that in its unsecured position the tip 8 of the piercing member is exposed outside the protection chamber 9 of the first member 10 exposing the tip 8 to any user operating the fluid transfer device.

Engagement means 60 are arranged on the first end 21 of the second member 20 to engage a first container 5 (shown schematically in fig. 1) to prevent the second member 20 from turning during connection.

An example of a first container 5 and its connection means is described in greater detail in WO 03/030809 A1. The engagement means 60 are in the form of a longitudinal protrusion extending in the direction of the longitudinal axis A which engages the first container 5 in a corresponding groove on the first container. It is however well within the boundaries of the present invention that the engagement means 60 may be constituted by a groove on the second member 20 which engages a corresponding protrusion on the first container 5. As an alternative the second member 20 may be held in place by the user during turning, in which case no engagement means are necessary.

Advantageously, the first end 11 of the first member 10 is equipped with a flexible barrier member. In may further be designed and arranged for creating a double-membrane sealing when the connection means 15 is connected to the first container 5. In such case the first container 5 may be e.g. an infusion bag of an infusion system, an infusion fluid line of the mentioned infusion system or a separate spike device exhibiting a flexible barrier member. Preferably, the first end 11 of the first member 10 is designed and arranged for all these cases. Double membrane bayonet couplings are known per se from the U. S. Patent No. 4,564, 054 for example and will hereafter not be described in greater detail. As a measure of safety a second flexible barrier member 17 may be provided at the second end 12 of the first member 10. The flexible barrier members 16, 17 are liquid and gas-proof sealing members which seal tightly around the piercing member to prevent leakage of liquid as well as gas contaminants. In cases where the piercing member is arranged on the second connection part 3 it preferably extends through the second flexible barrier member so its tip 8 is arranged inside protection chamber 9 of the first member 10.

As described the piercing member protection part 2 comprises of three members arranged together in working cooperation. It is however within the boundaries of the present invention that the piercing member protection part 2 may be designed in different ways.

As illustrated in fig. 1 the second connection part 3 comprises a turning grip 41 intended to aid a user to get a firm grip of the second connection part 3 in order to securely turn the second connection part 3. The second connection part 3 has a mounting part 42 arranged to the turning grip 41 which after assembly is rotably mounted to the first connection part 2, in this embodiment the third member 30. The mounting part 42 is at least partly arranged inside the third member 30. In this embodiment, the mounting part 42 comprises dual function locking means. A mounting means 44 prevents the second connection part 3 from sliding along the longitudinal axis A while at the same time allowing for a rotational movement, i.e. to be turned, of the second connection part 3 with respect first connection part 2, in this embodiment the piercing member protection part 2.

A first locking means 45 prevents the second connection part 3 from turning in a predetermined direction, which is either clock-wise or anti clock-wise, with respect to the first connection part 2. Connection means 4 for connection to a syringe or similar is arranged on the second connection part 3.

The second connection part 3 is mounted to the first connection part 2 by mounting means 44 comprising a locking groove 46 arranged transversally to the longitudinal axis A around the periphery of the mounting part 42. The locking groove interacts with at least one locking protrusion 33 arranged on the inside of the third member 30. Generally, the locking protrusion 33 is preferably slightly flexible so as to allow for an easy assembly of the third member 30 and the second connection part 3. When the locking protrusion 33 locks into the locking groove 46, and thereby the first connection part 2, it substantially locks the second connection part 3 from axial movement along the longitudinal axis A with respect to the first connection part 2, while at the same time, allowing for the second connection part 3 to be turned with respect to the first connection part 2.

It is to be understood that by substantially locking the second connection part 3 from axial movement along the longitudinal axis A with respect to the first connection part 2 does not necessarily mean a firm fixation of the second connection part 3 with respect to the first connection part 2. Instead the second connection part 3 may very well have a certain play with respect to the first connection part 2. Preferably such play allows the first connection part 2 to disengage with the second connection part 3 so that when the first locking means 45 are disengaged the first connection part 2 may be turned in any direction with respect to the second connections means 3. In this embodiment the first connection part 2 and the second connection part 3 has two positions, one in which the first locking means 45 is engaged so as to prevent the turning of the first connection part 2 with respect to the second connection part 3 in a predetermined direction and one position in which the first connection part 2 may be turned in any direction with respect to the second connection part 3.

Although the mounting means 44 has been described as a locking groove 46 it is within the boundaries of the present invention that any means which may provide the desired effect of locking the movement of the second connection part 3 along the longitudinal axis A while at the same time allowing the second connection part 3 to be turned in at least one direction with respect to the first connection part 2 may be suitable as mounting means 44.

In the illustrated embodiment in figures 1-3, the first locking means 45 comprise a plurality of saw tooth protrusions 47 arranged on the mounting part 42. The first connection part 2, and more specifically in the shown embodiment of the present invention, the third member 30 on the piercing member protection device, comprises an interacting protrusion element such as an interacting saw tooth protrusion (not shown in fig. 1) which after assembly with the second connection part 3 interacts with the plurality of saw tooth protrusions 47 on the second connection part 3 so that the second connection part is only able to turn in a clock-wise or an anti clock-wise direction after assembly. Although the first locking means 45 has been described as a saw tooth protrusion it is within the boundaries of the present invention that any means which may provide the desired effect of preventing the second connection part 3 from turning in one direction (e.g. in a clock-wise direction) while allowing for the second connection part 3 to be turned in an opposite direction (e.g. anti clock-wise direction) may be suitable as second locking means 45.

The mounting means 44 and the first locking means 45 are in the shown embodiment in fig. 1 illustrated as separate means. It is within the boundaries of the present invention that the mounting means 44 and the first locking means 45 are integrally formed.

The second connection part 3 further comprises a stabilization member 40 arranged to stabilize the piercing member 7. The stabilization member 40 extends through the third member 30 into the confined chamber defined by the first member 10 and the first and second flexible membrane barriers 16, 17 of the first member 10. The stabilization member 40 is preferably formed of a hollow tube which at least partly encloses the piercing member in order to prevent the piercing member from breaking or bending. The second connection part 3 is further equipped with connection means 4 for connection to a second fluid container.

In one embodiment of the present invention the stabilization member 40 may be arranged with said mounting means 44. When the stabilization member is in the form of a hollow tube, the hollow tube comprises an outer surface and a protrusion extending away from the longitudinal axis A on the outer surface of the hollow tube. In this case the protrusion preferably has an inclining surface so as to enable a simple connection to said first connection part 2.

In fig. 2 the fluid transferring device 1 is shown in its unsecured position, i.e. the tip 8 of the piercing member 7 is exposed to the environment. The piercing member 7 has pierced the flexible barrier member 16 in order to provide for a fluid communication between a first fluid container e.g. an infusion bag or a vial (attached to connection means 15 of the first member 10) and a second fluid container e.g. a syringe (attached to connection means 4 of the second connection part 3). When a fluid transfer device 1 as shown in fig. 1 and 2 is in its secured or unsecured position it is important to allow the second fluid container to be turned in order to easily see a volume indication or similar on the second container. The second connection part 3 enables this even in the unsecured position as shown in fig. 2.

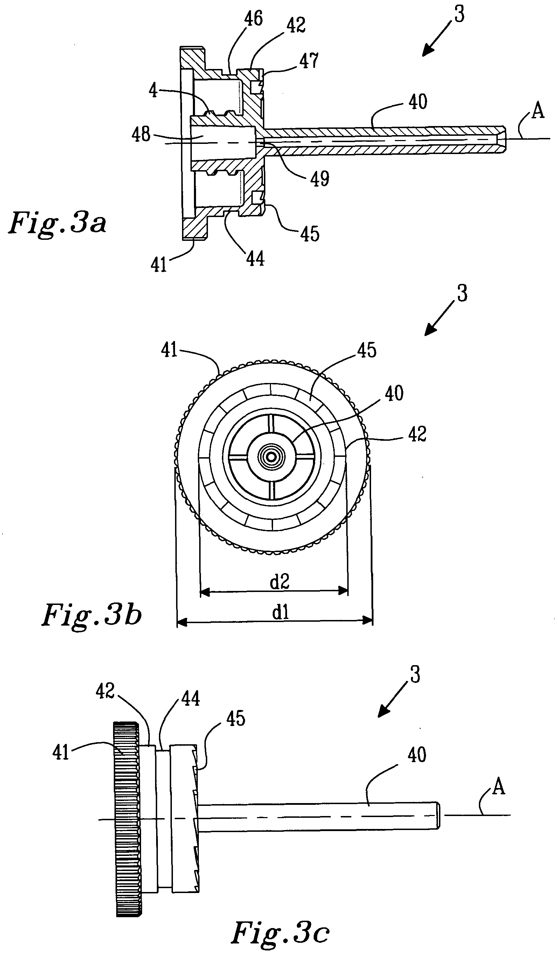

Fig. 3a shows a cross section of a second connection part 3 of the fluid transfer device according to the present invention. As mentioned, the second connection part 3 is equipped with connection means 4 for connection to a syringe or similar. The connection means 4 comprises a threaded coupling onto which e.g. a syringe may be screwed in order to provide a fluid flow through the second connection part 3 and a first connection part 2 when such is mounted. When screwing the second fluid container onto the threaded coupling the first locking means 45 engages the first connection part 2 (if there is a play) so as to lock the second connection part 3 from turning in a predetermined direction, i.e. the direction of which the second fluid container is screwed. Likewise after the second fluid container has been screwed onto the threaded coupling the second fluid container cannot (without much effort or actively prevention of the second connection part 3 from turning) be unscrewed because the first locking means 45 allows the second fluid container and the second connection part 3 to be turned in a direction opposite to the predetermined direction.

The second connection part 3 is preferably equipped with a stabilization member 40 arranged to stabilize a piercing member when such is arranged in the second connection part 3. The connection means 4 further defines an opening 48 into which a part of e.g. a syringe may be screwed. A fluid channel orifice 49 is arranged to provide for a fluid channel between the opening 48 and the piercing member 7. If no piercing member is arranged on the second connection part 3, a flexible barrier member (not shown) is advantageously arranged to cover the fluid channel orifice 49 in order to facilitate a safe environment for the user.

The second connection part 3 comprises a turning grip 41 intended to aid a user to get a firm grip of the second connection part 3 to securely turn the second connection part 3. Such a turning grip 41 is however not necessary as the second connection part 3 may be turned by means of the second fluid container after attachment with the second connection part 3. However, the turning grip 41 may advantageously be used to hold the second connection part 3 in a firm grip during disengagement with a second fluid container.

As described earlier a mounting part 42 is arranged to the turning grip 41, the mounting part 42 connects and holds the piercing member protection part after assembly. The mounting part 42 connects to the piercing member protection part by means of mounting means 44, illustrated in fig. 1 as a locking groove 46 arranged transversally to the longitudinal axis A around the periphery of the mounting part 42. The mounting part 42 is during assembly with a piercing member protection part of a fluid transfer device attached with at least one interacting locking protrusion 33 of the mentioned piercing member protection part (see fig. 1 and 2).

A first locking means 45 is arranged on the mounting part 42. The first locking means 45 is in fig 3a. illustrated as a plurality of saw tooth protrusions 47 which are arranged in a circle around the stabilization member 40. The plurality of saw tooth protrusions 47 interacts with interacting means on the first connection part 2, in the illustrated embodiment the piercing member protection part, so as to allow for a 360° turn of the second connection part in a predetermined direction. The interacting means may as mentioned be interacting saw tooth elements, however, any kind of means able to interact with a saw tooth protrusions 47 in order to allow for a 360° turn of the second connection part 2 in a predetermined direction may be used. The first locking means 45 is preferably designed for a clock-wise turn or an anti clock-wise turn of the second connection part 3, its however within the boundaries of one embodiment of the present invention that the first locking means 45 allows for a step-wise turning in both a clock-wise and/or an anti clock-wise direction of the second connection part 3. Such step-wise turning is advantageous since it induces a moment of inertia to the second connection part 3 which prevents a connected syringe or similar from turning too easily. The first locking means 45 has in such an embodiment of the present invention a plurality of e.g. sinusoidal shaped protrusions (wave like) more than saw tooth shaped protrusions as described earlier. Of course the piercing member protection part is equipped with corresponding means in order to facilitate such controlled turning of the second connection part.

Fig. 3b shows the second connection part 3 as seen along the longitudinal axis A. The first locking means 45 circles the stabilization means 40 in order to provide for a 360° turn of the second connection part. The second connection part 3 has in the shown embodiment of the present invention a circular cross section. The turning grip has a diameter d₁ while the mounting part 42 has a diameter d₂. In the shown embodiment of fig. 3b d₂ is smaller than the inner diameter of the third member 30 in order to facilitate the assembly of the second connection part 3 into the third member 30 of first connection part 2 of the fluid transfer device 1 as described earlier. This is further illustrated in fig. 3c in which a side view of the second connection part of the present invention is shown. Further are a mounting means 44 and a first locking means 45 arranged on the mounting part 42 which in turn is arranged to a turning grip 41. Stabilization means 40 extends along the longitudinal axis A out from the centre of the connection means 42. In the shown embodiment of the present invention the turning grip 41, the connection means 42 and the stabilization means 40 are formed in one piece of material, it is however within the boundaries of the present invention that the turning grip 41, the mounting means 42 and the stabilization means 40 are formed of separate pieces of material attached to each other.

The first member of the piercing member protection device according to one embodiment of the present invention has preferably a cylindrical inside, but more preferably, to simplify the manufacturing, it's a cylinder member. Likewise is the second and third member preferably cylinder members.

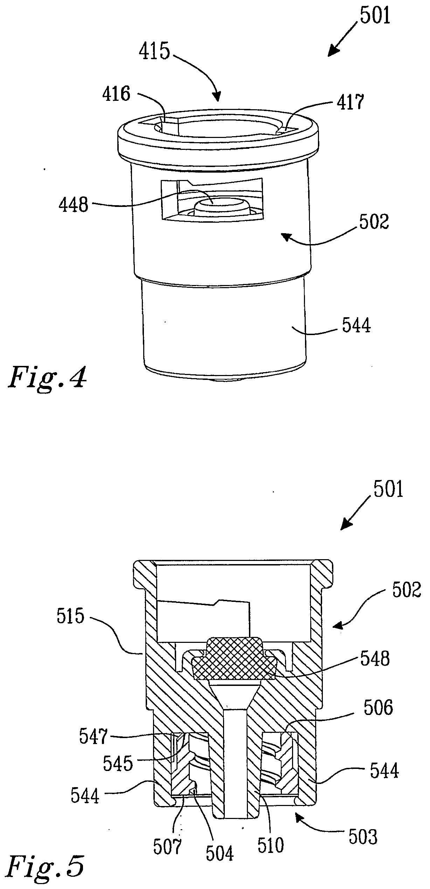

Fig. 4 shows a fluid transfer device 501 according to an embodiment of the present invention. The fluid transfer device 501 has connection means 415 having a neck element comprising two guiding members 416, 417 to which a first fluid container, such as an injector, may be connected. The first connection part 502 is seen provided with mounting means 544 which by the second connection part is mounted to the first connection part 502. A barrier member 448 can be seen through the neck element of the connection means 415.

Fig. 5 is a cross section of the embodiment shown in fig. 4 of the present invention. Fig. 5 shows the cross section of a fluid transfer device 501 according to the present invention in the form of a connector having a first connection part 502 with a cylinder like form which is rotably mounted to a second connection part 503 via mounting means 544. The mounting means 544 substantially prevents the second connection part 503 from sliding along the longitudinal axis A while at the same time allowing for a rotational movement, i.e. to be turned, of the second connection part 503 with respect to the first connection part 502. In the shown embodiment mounting means 544 comprises a cylinder shaped extension of the first connection part 502 which at least partly encloses the second connection part 503. However it does not enclose the second connection part 503 so as to occlude the connection means 504. As seen in fig. 5 the first connection part 502 comprise connection means 515 to connect to a first fluid container, preferably an injector e.g. as described in WO 2004/004806 (injection component 29).

A first locking means 545, as described in earlier embodiments, prevents the second connection part 503 from turning in a predetermined direction, which is either clock-wise or anti clock-wise, with respect to the first connection part 502. As in the earlier embodiments the first locking means 545 may comprise a plurality of saw tooth protrusions which are arranged in a circle on the second connection part 503. The plurality of saw tooth protrusions interacts with interacting means on the first connection part 502, so as to allow for at least a 90°, more preferably at least a 180°, or even more preferably a 270° or most preferably at least a 360° turn of the second connection part in a predetermined direction.
Connection means 504, in the form of a threaded coupling, preferably forming part of a luer-lock coupling, for connection to an infusion bag or similar is arranged on the second connection part 503. In the shown embodiment of the present invention the second connection part 503 has substantially the shape of a rotatable cylinder having a threaded coupling arranged on the inside of the cylinder (i.e. the inner surface) while having a smooth surface on the outer surface of the cylinder. The second connection part 503 has a first and a second end 506, 507. The first end 506 is arranged towards the first connection part 502 while the second end 507 of the second connection part 503 is arranged facing away from the first connection part 502. The second connection part 503 is encircling a conical shaped protrusion 510 extending out from the first connection part 502, the conical shaped protrusion 510 forms together with the threaded coupling part of a luer-lock coupling.

The second connection part 503 may have a play with respect to the first connection part 502. Preferably such play allows the first connection part 502 to partly disengage with the second connection part 503 so that the first locking means 545 is disabled. The first connection part 502 may then be turned in any direction with respect to the second connections means 503. In this embodiment the first connection part 502 and the second connection part 503 have two positions, one in which the first locking means 545 is engaged so as to prevent the turning of the first connection part 502 with respect to the second connection part 503 in a predetermined direction and one position in which the first connection part 502 may be turned in any direction with respect to the second connection part 503.

If as shown in fig. 5, the second connection part 503 comprises of an open ended cylinder, i.e. the first end 506 of the second connection part 503 is open towards the first connection part 502, and the mounting means 544 is made preferably 3-15 % longer than the second connection part 503. The second connection part 503 will when threaded onto a fluid container be unable to engage the first locking means 545 again after disengagement of the first locking means 545. This is due to that the fluid container is allowed to maintain the play since it will run through the second connection part 503 and displace the second connection part 503 towards to first connection part 502. In this sense, a permanent disengagement of the first locking means is achieved. Once the first locking means 545 is disengaged it can not again engage since no matter how the second connection means 503 is turned the first locking means 545 will not engage again.

In the illustrated embodiment in figure 5, the first locking means 545 comprise a plurality of saw tooth protrusions 547 (as described earlier in fig. 1-3). The first connection part 502 comprises an interacting protrusion element such as an interacting saw tooth protrusion (not shown in fig. 5) which after assembly with the second connection part 503 interacts with the plurality of saw tooth protrusions 547 on the first end 506 of the second connection part 503 so that the second connection part is only able to turn in a clock-wise or an anti clock-wise direction after engagement. Although the first locking means 545 has been described as a protrusion such as a saw tooth protrusion, and interacting protrusion element, it is within the boundaries of the present invention that any means which may provide the desired effect of preventing the first connection part 502 from turning in a predetermined direction (e.g. in a clock-wise direction) with respect to the second connection part 503 may be suitable as first locking means 545 while still allowing it to turn in a direction opposite of the predetermined direction.

The mounting means 544 and the first locking means 545 are in the shown embodiment in fig. 1-5 illustrated as separate means. It is within the boundaries of the present invention that the mounting means 544 and the first locking means 545 are integrally formed. The first locking means 545 may for instance be arranged on the mounting means 544.

A flexible barrier member 548 is arranged in the first connection part 502. The flexible barrier member 548 is a liquid and gas-proof sealing member which may seal tightly around a piercing member to prevent leakage of liquid as well as gas contaminants before as well as after insertion or retraction of the piercing member. A piercing member may be arranged either as described earlier to the fluid transfer device 501, or as a separate device, e.g. on a fluid container such as a syringe, which can be arranged to the fluid transfer device 501.

## Claims

1. A fluid transfer device for transferring fluid from a first fluid container to a second fluid container, said fluid transfer device (1) comprises a longitudinal axis (A), a first connection part (2) comprising a connector (15) that connects to said first fluid container,
said fluid transfer device (1) further comprises a second connection part (3, 503) rotatably mounted to said first connection part (2) by a mounting element (44), said second connection part (3) comprises a threaded coupling element (4) that connects said second fluid container,
said fluid transfer device (1) is further arranged with at least one protrusion (45), wherein said at least one protrusion (45) substantially prevents said first connection part (2) from turning in a predetermined direction with respect to said second connection part (3) while still allowing it to turn in a direction opposite of
said predetermined direction,
**characterized in**
**that** said device is a piercing member protection device (1) comprising a piercing member protection part (2) having a protection chamber (9) to protect a piercing member (7), said piercing member protection device (1) having a secured position, in which at least the tip (8) of said piercing member (7) is enclosed within said protection chamber (9) of said piercing member protection part (2), so as to prevent said tip (8) of said piercing member (7) from exposure,
and an unsecured position, in which said tip (8) of the piercing member (7) is arranged outside the protection chamber (9) of said piercing member protection part (2).

2. The fluid transfer device according to any of claim 1 **characterized in that** said at least one protrusion (45) on said fluid transfer device (1) enables at least a 90° turn, preferably at least a 180° turn, more preferably at least a 270° turn, or even more preferably at least a 360° turn of said second connection part (3).

3. The fluid transfer device according to any of claims 1 or claim 2 **characterized in that** said mounting element (44) comprises a locking groove (46) arranged transversally to the longitudinal axis (A) around the periphery of a mounting part that connects to said piercing member protection part (2).

4. The fluid transfer device according to claim 1 **characterized in that** said fluid transfer device (1) further comprises a stabilization member (40) arranged to stabilize said piercing member (7).

5. The fluid transfer device according to claim 1 **characterized in that** said stabilization member (40) comprises a hollow tube arranged along the longitudinal axis A or parallel thereto, wherein said hollow tube is arranged to at least partly enclose said piercing member (7).

6. The fluid transfer device according to any of claims 1 to 5, wherein said at least one protrusion engages at least one interacting protrusion element on said first connection part (2).

7. The fluid transfer device according to claim 1 **characterized in that** said piercing member protection device (1) is a medical piercing member protection device.

8. The fluid transfer device according to any of claims 1 to 7 **characterized in that** the first connection part (2) and the second connection part (3) have two positions, one in which the at least one protrusion (45) is engaged so as to prevent the turning of the first connection part (2) with respect to the second connection part (3) in a predetermined direction, and one position in which the first connection part (2) may be turned in any direction with respect to the second connection part (3).

9. The fluid transfer device according to claim 7 **characterized in that** said two positions are enabled by a play between the first connection part (2, 502) and the second connection part (3).

10. The fluid transfer device according to any of claims 1 to 9 **characterized in that** said threaded coupling element (4) on said second connection part (3) interacts with said at least one protrusion (45) so that said predetermined direction in which the first connection part (2) is prevented from turning with respect to said second connection part (3) is the same direction as the second connection part (3) is turned in order to connect a second fluid container thereto.

11. The fluid transfer device according to any of claims 1 to 10 **characterized in that** said connector (15) on said first connection part (2, 502) interacts with said at least one protrusion (45) so that said predetermined direction in which the first connection part (2) is prevented from turning with respect to said second connection part (3) is the same direction as the second connection part (3) is turned in order to connect a first fluid container thereto.

12. The fluid transfer device according any preceding claims **characterized in that** said first connection part (2) is provided with a barrier member.

## Patentansprüche

1. Flüssigkeitstransfervorrichtung für den Transfer einer Flüssigkeit von einem ersten Flüssigkeitsbehälter zu einem zweiten Flüssigkeitsbehälter, wobei die Flüssigkeitstransfervorrichtung (1) eine Längsachse (A), ein erstes Verbindungsteil (2) mit einem Anschluss (15) aufweist, der dem Anschluss an den ersten Flüssigkeitsbehälter dient,
wobei die Flüssigkeitstransfervorrichtung (1) des Weiteren ein zweites Verbindungsteil (3, 503) aufweist, das mittels eines Befestigungselements (44) drehbar an dem ersten Verbindungsteil (2) angebracht ist, wobei das zweite Verbindungsteil (3) ein Kopplungselement (4) mit Gewinde besitzt, das mit dem zweiten Flüssigkeitsbehälter verbindbar ist,
wobei die Flüssigkeitstransfervorrichtung (1) außerdem mit mindestens einem Vorsprung (45) ausgebildet ist, wobei der mindestens eine Vorsprung (45) im Wesentlichen verhindert, dass sich das erste Verbindungsteil (2) in einer vorgegebenen Richtung bezüglich des zweiten Verbindungsteils (3) dreht, während immer noch eine Drehung in einer Richtung zugelassen wird, die der vorgegebenen Richtung entgegengesetzt ist,
**dadurch gekennzeichnet,**
**dass** die Vorrichtung eine Schutzvorrichtung (1) für ein Einstechteil ist, welche ein Schutzteil (2) für das Einstechteil mit einer Schutzkammer (9) zum Schutz eines Einstechteils (7) besitzt, wobei die Schutzvorrichtung für das Einstechteil (1) eine gesicherte Stellung besitzt, in welcher zumindest die Spitze (8) des Einstechteils (7) im Inneren der Schutzkammer (9) des Schutzteils (2) für das Einstechteil so eingeschlossen ist, dass ein Freiliegen der Spitze (8) des Einstechteils (7) verhindert wird, und dass das Einstechteil außerdem eine ungesicherte Position besitzt, in welcher die Spitze (8) des Einstechteils (7) außerhalb der Schutzkammer (9) des Schutzteils (2) für das Einstechteil angeordnet ist.

2. Flüssigkeitstransfervorrichtung nach Anspruch 1, **dadurch gekennzeichnet, dass** der mindestens eine Vorsprung (45) auf der Flüssigkeitstransfervorrichtung (1) eine Drehung des zweiten Verbindungsteils (3) um mindestens 90° zulässt, vorzugsweise um mindestens 180°, besonders bevorzugt um mindestens 270°, oder ganz besonders bevorzugt um mindestens 360° zulässt.

3. Flüssigkeitstransfervorrichtung nach Anspruch 1 oder Anspruch 2, **dadurch gekennzeichnet, dass** das Befestigungselement (44) eine Arretierungsnut (46) aufweist, die quer zur Längsachse (A) um den Umfang eines Befestigungsteils herum angeordnet ist, welches für den Anschluss an das Schutzteil (2) für das Einstechteil vorgesehen ist.

4. Flüssigkeitstransfervorrichtung nach Anspruch 1, **dadurch gekennzeichnet, dass** die Flüssigkeitstransfervorrichtung (1) des Weiteren ein Stabilisierungselement (40) aufweist, das zur Stabilisierung des Einstechteils (7) angeordnet ist.

5. Flüssigkeitstransfervorrichtung nach Anspruch 1, **dadurch gekennzeichnet, dass** das Stabilisierungselement (40) ein hohles Rohrteil aufweist, das entlang der Längsachse A oder parallel zu dieser angeordnet ist, wobei das hohle Rohrteil so angeordnet ist, dass es das Einstechteil (7) zumindest teilweise einschließt.

6. Flüssigkeitstransfervorrichtung nach einem der Ansprüche 1 bis 5, bei welcher der mindestens eine Vorsprung mit mindestens einem damit zusammenwirkenden vorspringenden Element auf dem ersten Verbindung steil (2) in Eingriff steht.

7. Flüssigkeitstransfervorrichtung nach Anspruch 1, **dadurch gekennzeichnet, dass** die Schutzvorrichtung (1) für das Einstechteil eine Schutzvorrichtung für Einstechteile für medizinische Zwecke ist.

8. Flüssigkeitstransfervorrichtung nach einem der Ansprüche 1 bis 7, **dadurch gekennzeichnet, dass** das erste Verbindungsteil (2) und das zweite Verbindungsteil (3) zwei Positionen besitzen, und zwar eine, in welcher der mindestens eine Vorsprung (45) so in Eingriff steht, dass er eine Drehung des ersten Verbindungsteils (2) bezüglich des zweiten Verbindungsteils (3) in einer vorgegebenen Richtung verhindert, und eine zweite Position, in welcher das erste Verbindungsteil (2) bezüglich des zweiten Verbindungsteils (3) in jede Richtung gedreht werden kann.

9. Flüssigkeitstransfervorrichtung nach Anspruch 7, **dadurch gekennzeichnet, dass** die beiden Positionen durch ein Spiel zwischen dem ersten Verbindungsteil (2, 502) und dem zweiten Verbindungsteil (3) ermöglicht werden.

10. Flüssigkeitstransfervorrichtung nach einem der Ansprüche 1 bis 9, **dadurch gekennzeichnet, dass** das Kopplungselement (4) mit Gewinde auf dem zweiten Verbindungsteil mit dem mindestens einen Vorsprung (45) so zusammenwirkt, dass die vorgegebene Richtung, in welcher eine Drehung des ersten Verbindungsteils (2) bezüglich des zweiten Verbindungsteils (3) verhindert wird, die gleiche Richtung ist wie jene, in die sich das zweite Verbindungsteil (3) für den Anschluss eines zweiten Flüssigkeitsbehälters daran dreht.

11. Flüssigkeitstransfervorrichtung nach einem der Ansprüche 1 bis 10, **dadurch gekennzeichnet, dass** der Anschluss (15) auf dem ersten Verbindungsteil (2, 502) mit dem mindestens einen Vorsprung so zusammenwirkt, dass die vorgegebene Richtung, in welcher eine Drehung des ersten Verbindungsteils (2) bezüglich des zweiten Verbindungsteils (3) verhindert wird, die gleiche ist wie jene, in welche sich das zweite Verbindungsteil (3) für den Anschluss eines ersten Flüssigkeitsbehälters daran gedreht wird.

12. Flüssigkeitstransfervorrichtung nach einem der vorhergehenden Ansprüche, **dadurch gekennzeichnet, dass** das erste Verbindungsteil (2) mit einem Sperrenteil versehen ist.

## Revendications

1. Dispositif de transfert de fluide destiné à transférer un fluide depuis un premier conteneur de fluide vers un second conteneur de fluide, ledit dispositif de transfert de fluide (1) comprend un axe longitudinal (A), une première partie de liaison (2) comprenant un raccord (15) qui est raccordé audit premier conteneur de fluide,
ledit dispositif de transfert de fluide (1) comprend en outre une seconde partie de liaison (3, 503) montée de manière à pouvoir tourner sur ladite première partie de liaison (2) par un élément de montage (44),
ladite seconde partie de liaison (3) comprend un élément de couplage fileté (4) qui est relié audit second conteneur de fluide,
ledit dispositif de transfert de fluide (1) comporte en outre au moins une saillie (45),
dans lequel ladite au moins une saillie (45) empêche sensiblement ladite première partie de liaison (2) de tourner dans un sens prédéterminé par rapport à ladite seconde partie de liaison (3) tout en lui permettant toujours de tourner dans un sens opposé audit sens prédéterminé,
**caractérisé en ce que** ledit dispositif est un dispositif de protection d'élément de perçage (1) comprenant une partie de protection d'élément de perçage (2) comportant une chambre de protection (9) pour protéger un élément de perçage (7), ledit dispositif de protection d'élément de perçage (1) présentant une position sécurisée, dans laquelle au moins l'extrémité (8) dudit élément de perçage (7) est enfermée à l'intérieur de ladite chambre de protection (9) de ladite partie de protection d'élément de perçage (2), de manière à empêcher l'exposition de ladite pointe (8) dudit élément de perçage (7),
et une position non sécurisée, dans laquelle ladite pointe (8) de l'élément de perçage (7) est agencée à l'extérieur de la chambre de protection (9) de ladite partie de protection d'élément de perçage (2).

2. Dispositif de transfert de fluide selon la revendication 1, **caractérisé en ce que** ladite au moins une saillie (45) sur ledit dispositif de transfert de fluide (1) permet au moins une rotation à 90°, de préférence, au moins une rotation à 180°, plus préférablement, au moins une rotation à 270°, ou encore plus préférablement, au moins une rotation à 360° de ladite seconde partie de liaison (3).

3. Dispositif de transfert de fluide selon l'une quelconque des revendications 1 ou 2 **caractérisé en ce que** ledit élément de montage (44) comprend une rainure de verrouillage (46) agencée transversalement par rapport à l'axe longitudinal (A) autour de la périphérie d'une partie de montage qui relie ladite partie de protection d'élément de perçage (2).

4. Dispositif de transfert de fluide selon la revendication 1, **caractérisé en ce que** ledit dispositif de transfert de fluide (1) comprend en outre un élément de stabilisation (40) agencé de manière à stabiliser ledit élément de perçage (7).

5. Dispositif de transfert de fluide selon la revendication 1, **caractérisé en ce que** ledit élément de stabilisation (40) comprend un tube creux agencé le long de l'axe longitudinal A ou parallèlement à celui-ci, dans lequel ledit tube creux est agencé de manière à envelopper partiellement ledit élément de perçage (7).

6. Dispositif de transfert de fluide selon l'une quelconque des revendications 1 à 5, dans lequel ladite au moins une saillie est couplée à au moins un élément en saillie coopérant sur ladite première partie de liaison (2).

7. Dispositif de transfert de fluide selon la revendication 1, **caractérisé en ce que** ledit dispositif de protection d'élément de perçage (1) est un dispositif de protection d'élément de perçage médical.

8. Dispositif de transfert de fluide selon l'une quelconque des revendications 1 à 7, **caractérisé en ce que** la première partie de liaison (2) et la seconde partie de liaison (3) comportent deux positions, une première dans laquelle la au moins une saillie (45) est couplée de manière à empêcher la rotation de la première partie de liaison (2) par rapport à la seconde partie de liaison (3) dans un sens prédéterminé, et une position dans laquelle la première partie de liaison (2) peut être tournée dans un sens quelconque par rapport à la seconde partie de liaison (3).

9. Dispositif de transfert de fluide selon la revendication 7, **caractérisé en ce que** lesdites deux positions sont validées par un jeu entre la première partie de liaison (2, 502) et la seconde partie de liaison (3).

10. Dispositif de transfert de fluide selon l'une quelconque des revendications 1 à 9 **caractérisé en ce que** ledit élément de couplage fileté (4) sur ladite seconde partie de liaison (3) coopère avec ladite au moins une saillie (45) de telle sorte que ledit sens prédéterminé dans lequel la première partie de liaison (2) est empêchée de tourner par rapport à ladite seconde partie de liaison (3) est le même sens que celui dans lequel la seconde partie de liaison (3) est tournée dans le but d'être raccordée à un second conteneur de fluide.

11. Dispositif de transfert de fluide selon l'une quelconque des revendications 1 à 10, **caractérisé en ce que** ledit raccord (15) sur ladite première partie de liaison (2, 502) coopère avec ladite au moins une saillie (45) de telle sorte que ledit sens prédéterminé dans lequel la première partie de liaison (2) est empêchée de tourner par rapport à ladite seconde partie de liaison (3) est le même sens que celui dans lequel la seconde partie de liaison (3) est tournée dans le but d'être raccordée à un premier conteneur de fluide.

12. Dispositif de transfert de fluide selon l'une quelconque des revendications précédentes, **caractérisé en ce que** ladite première partie de liaison (2) comporte un élément barrière.
